# EUROPEAN PATENT APPLICATION

(11) **EP 4 512 476 A2**
(43) Date of publication of application: **26.02.2025**
(21) Application number: 24206877.3
(22) Date of filing: 16.11.2017
(51) Int. Cl.: A61P 33/10

(54) **USE OF CELL MEMBRANE-BOUND SIGNALING FACTORS**

(30) Priority: 16.11.2016 US 201662422900 P
(62) Divisional of application: 17870876.4
(71) Applicant: Aivita Biomedical, Inc., Irvine, CA 92612 (US)
(72) Inventor: NISTOR, Gabriel, Irvine, 92612 (US)
(74) Representative: Mewburn Ellis LLP

(57) **Abstract**

Disclosed herein are compositions comprising complexes of cyclodextrins and lipid- modified stem cell proteins. Also disclosed are topical compositions the complexes. Methods of using the compositions for the therapeutic purposes are also disclosed as well as methods of producing the compositions.

## Description

### CROSS REFERENCE TO RELATED APPLICATIONS

The present application claims the benefit under 35 U.S.C. §119(e) to U.S. provisional patent application 62/422,900 filed November 16, 2016, the entire contents of which are incorporated by reference herein.

### BACKGROUND

Membrane bound signaling factors, including proteins of the Wingless (Wnt) and Hedgehog (Hh) families have the potential for use in a variety of disorders, however current methods of obtaining these proteins do not yield stable, efficacious molecules.

### SUMMARY

Disclosed herein are compositions comprising a complex of lipid-modified proteins and a cyclodextrin as disclosed herein.

Also disclosed herein are topical compositions comprising the complex of lipid-modified proteins and a cyclodextrin as disclosed herein.

In some embodiments, the cyclodextrin is one or more of α-cyclodextrin, β-cyclodextrin, γ-cyclodextrin, or methyl-β-cyclodextrin. In some embodiments, the cyclodextrin is a chemically modified cyclodextrin, modified by hydrogenation, hydroformylation, methylation, oxidation, reduction, or a carbon-carbon coupling reaction. In some embodiments, the cyclodextrin is methyl-β-cyclodextrin.

In some embodiments, the lipid-modified proteins comprise one or more Wingless (Wnt) or Hedgehog (Hh) proteins associated with a cell membrane lipid. In some embodiments, the Hh protein is one or more of a Sonic Hedgehog (Shh) protein, a Desert Hedgehog (Dhh) protein, or an Indian Hedgehog (Ihh) protein. In some embodiments, the Wnt protein is one or more of Wnt3a, Wnt7b, or Wnt10b. In some embodiments, the lipid-modified proteins comprise of other proteins than those belonging to the Wingless (Wnt) or Hedgehog (Hh) families.

In some embodiments, the lipid-modified proteins are harvested from a population of stem cells. In some embodiments, the stem cells are embryonic stem cells, parthenogenic stem cells, adult stem cells, fetal stem cells, or induced pluripotent stem cells. In some embodiments, the stem cells are mammalian stem cells. In some embodiments, the stem cells are human stem cells. In some embodiments, the stem cells are genetically engineered to overexpress Wnt or Hh proteins. In some embodiments, the stem cells are generically engineered to be immortal. In some embodiments, the stem cells are genetically engineered to express telomerase reverse transcriptase (hTERT)

In some embodiments, the composition further comprises at least one kosmotrope. In some embodiments, the at least one kosmotrope is propylene glycol, proline, trehalose, ectoine, or trimethylamine N-oxide.

In some embodiments, the topical composition is in an aqueous formulation. In some embodiments, the topical composition further comprises at least one kosmotrope, and an antimicrobial agent. In some embodiments, the at least one kosmotrope is trehalose. In some embodiments, the antimicrobial agent comprises silver particles. In some embodiments, the silver particles are silver nanoparticles or silver microparticles. In some embodiments, the pH of the topical composition is between about 4.5 and about 8.0.

Also disclosed herein are methods of promoting tissue regeneration in a tissue in need thereof, comprising exposing a tissue to a composition or topical composition disclosed herein.

Also disclosed herein are methods of promoting tissue rejuvenation in a tissue in need thereof, comprising exposing a tissue to a composition or topical composition disclosed herein.

Also disclosed herein are methods of restoring sensory nerve function in a tissue in need thereof, comprising exposing the tissue to a composition or topical composition disclosed herein.

In some embodiments, the tissue is skin. In some embodiments, the tissue is scar tissue.

Also disclosed herein are methods of promoting hair growth, comprising exposing hair follicles to a composition or topical composition disclosed herein. Also disclosed herein are methods of improving the appearance of hair, comprising exposing the hair follicles to a composition or topical composition disclosed herein. In some embodiments, the hair growth is promoted in a subject having senescent alopecia, alopecia totalis, tellogen and anagen effluvium, or alopecia areata. In some embodiments, the hair follicles are on the scalp of a subject. In some embodiments, the hair follicles are the eyelashes of a subject. In some embodiments, the hair follicles are the eyebrows of a subject. In some embodiments, the hair follicles are on the face of a subject. In some embodiments, the hair follicles are on the chest of a subject. In some embodiments, the hair follicles are on the arms of a subject. In some embodiments, the hair follicles are on the legs of a subject.

Also disclosed herein are methods of improving the appearance of skin, comprising exposing the skin to a composition or topical composition disclosed herein. In some embodiments, the appearance improved is one or more of skin texture, wrinkles, discolorations, and age spots

Also disclosed herein are methods of producing a composition or topical composition disclosed herein comprising: culturing stem cells which are capable of producing Wnt and Hh proteins in a culture media; incubating the cells in a harvest solution comprising a cyclodextrin to obtain cyclodextrin complexes of lipid-modified proteins; and mixing the cyclodextrin/lipid-modified protein complexes with one or more pharmaceutically acceptable excipients to form a topical formulation.

In some embodiments, the harvest solution further comprises at least one kosmotrope. In some embodiments, the harvest solution comprises a stabilizing agent. In some embodiments, the stabilizing agent is a kosmotrope. In some embodiments, the kosmotrope is trehalose. In some embodiments, the concentration of kosmotrope in the harvest solution is about 5% to about 30%. In some embodiments, the concentration of kosmotrope is 20%.

In some embodiments, the harvest solution comprises an aqueous solution of a cyclodextrin. In some embodiments, the cyclodextrin is methyl-β-cyclodextrin. In some embodiments, the concentration of cyclodextrin in the harvest solution is about 1 mM to about 20 mM. In some embodiments, the concentration of cyclodextrin in the harvest solution is about 10 mM.

In some embodiments, the cyclodextrin/lipid-modified protein complex solution is stored at 4°C or lower. In some embodiments, the cyclodextrin/lipid-modified protein complexes solution is lyophilized. In some embodiments, the one or more pharmaceutically acceptable excipients comprises one or more preservatives. In some embodiments, the one or more pharmaceutically acceptable excipients comprises one or more antimicrobial agents.

### BRIEF DESCRIPTION OF THE DRAWINGS

Figure 1 depicts the chemical and physical structure of cyclodextrins.
Figure 2 depicts a schematic representation of the trehalose effect on stabilizing lipid membranes during dehydration.
Figure 3 depicts interactions between local and regional factors in morphogenesis and tissue growth.
Figures 4A-B depict a schematic mechanism of membrane-bound lipid-modified protein capture using cyclodextrins. Figure 4A depicts that cyclodextrins in solution can capture the lipid-modified proteins attached to the cell membrane. Figure 4B depicts that cyclodextrin captured complexes can be further enhanced with the addition of trehalose that has a kosmotrope effect by displacing water surrounding the protein molecule and allowing a lyophilized storage method.
Figure 5A depicts cell cultures before exposure which were smooth compact and multilayered. Figure 5B depicts cultures after incubation with cyclodextrin wherein the cultures were disrupted with a majority of the cells losing adherence.
Figures 6A-D depict that mice in the treatment groups 1 (Figure 6A), 2 (Figure 6B), 3 (Figure 6C), and 4 (Figure 6D) demonstrated early transition to anagen by the presence of new anagen patches "anagen waves."
Figure 7A depicts that treated animals displayed a hair growth response, regardless of the active concentration group. Figure 7B depicts that treated animals displayed increased number of new anagen hair patches. Figure 7C depicts that the response to treatment was confirmed by increased darkness of the skin in the treatment groups.
Figures 8A-C depicts telogen status of hair follicles in treated mice. A sample was obtained at day one of the study (D1) to confirm that the mice are in the first telogen phase. The mice in the control group (Group 1, Figure 8A) maintained the telogen (T) status of the hair follicles at the end of the study (D20) (Figures 8B and C).
Figure 9A-C depicts telogen status of hair follicles in treated mice. The mice in the treatment groups (Groups 2, 3 and 4; Figures 9A, 9B, and 9C, respectively) displayed a mix of telogen and early anagen (EA) follicles in the area that was not containing new anagen patches.
Figure 10A-C depicts hair growth in treated mice. The mice in all treatment groups (Groups 2, 3 and 4; Figures 10A, 10B, and 10C, respectively) group displayed patches of new anagen with typical anagen hair follicle morphology.
Figure 11A-B depicts hair growth in two untreated mice. In control animals, the telogen stage persists with minimal or no Wnt activation, evidenced by very few LRG5 positive cells.

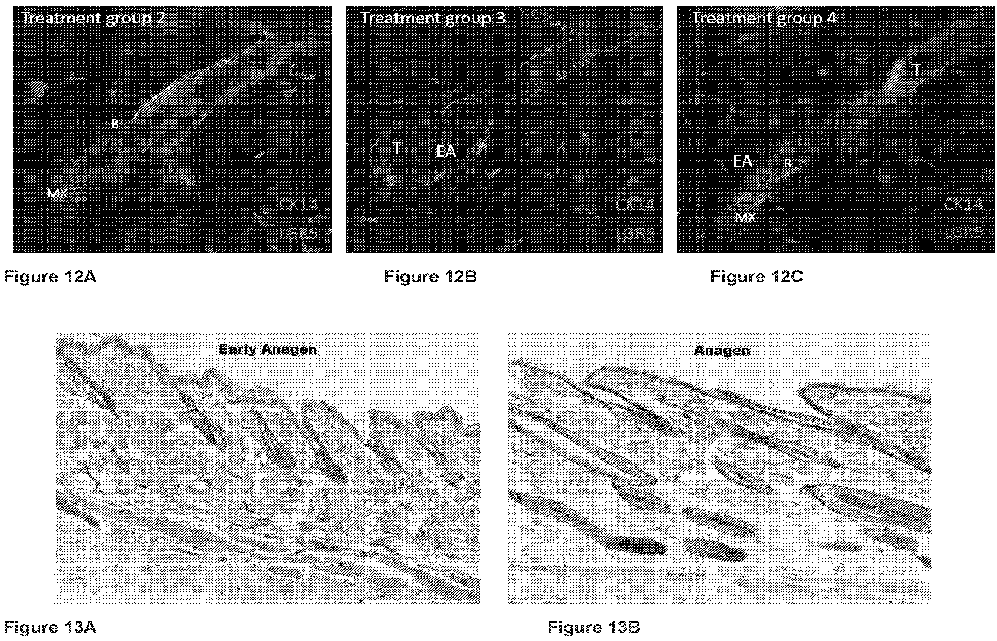

Figure 12A-C depicts hair follicles in treated mice. Figure 12A depicts treatment group 2 and is a photomicrograph of a new follicle. Expanding LGR5 positive bulge cells (B) migrating downwards and populating the new bulb matrix (MX). Figure 12B depicts treatment group 3 and is a photomicrograph of a new anagen follicle. Expanding LGR5 positive cells migrating downwards and populating the early anagen bulb (EA) next to a follicle in telogen (T). Figure 12C depicts treatment group 4 and is a photomicrograph of new anagen follicle. New follicle (EA) is below an old telogen follicle (T). LGR5 positive cells expanding the bulge area (B) and populating downwards the new bulb matrix (MX).
Figure 13A-B depicts hair growth in treated animals. The treated animals show hair stem cell mobilization by Sox9 positivity. Figure 13A depicts early anagen phase and Figure 13B depicts anagen phase.
Figures 14A-G depict the effects of methyl-β-cyclodextrin (MBCD) on the *in vitro* growth of hair follicles in the absence of other growth factors at various concentrations after 2 days (Figures 14A-C) and 5 days (Figures 14D-G) of culture.
Figure 15A depicts hair follicles grown in 0.25 mM MBCD complex displaying enhanced growth in length. Figure 15B depicts hair follicles grown in 0.25 mM and 0.5 mM MBCD complex displaying a statistical significant enhanced growth in thickness (p<0.01 for 0.25 mM and p<0.05 for the 0.5 mM group)
Figure 16A-D depicts the testing on human skin of a composition containing MBCD loaded with embryonic stem cell membrane components and trehalose. Figures 16A and 16C depict an untreated area. Figures 16B and 16D depict a treated contralateral area with visible restored velus hair, longer velus hair, and reduced aging spots.

### DETAILED DESCRIPTION

Although Wingless (Wnt) and Hedgehog (Hh) proteins have been isolated and characterized, there is a very limited application of these factors. The sources of these proteins commonly are cells engineered to overexpress one single particular protein which is removed from the cell surface with mild detergents.

Disclosed herein are complexes of lipid-modified proteins from stem cells and cyclodextrins. As used herein, the term "lipid-modified" refers to proteins having lipids covalently attached thereto. Regarding Wnt and Hh, the proteins are modified with the fatty acid palmitate, although modification with other lipids, including cholesterol is within the scope of the presently disclosed compositions and methods.

Stem cells that represent a transitional state from pluripotency to germ layer stages express significant quantities of Wnt and Hh proteins. However, although the culture supernatants contain numerous soluble growth factors, Wnt and Hh proteins are not identifiable in the cell culture supernatant. Physiologic expression of Wnt and Hh leads to modification with lipids, causing them to become associated with the surface membrane of the expressing cell, rather than their secretion into the extracellular fluid. By exposing stem cells to a cyclodextrin solution, it is possible to successfully extract the lipid-modified Wnt and Hh proteins bound to the cholesterol-containing cell membrane, thus forming a soluble complex of the lipid-modified protein bound to the cyclodextrin. Adding trehalose to the soluble lipid-modified protein/cyclodextrin complex leads to the stabilization of the complex, allowing long-term storage of a lyophilized complex.

The Wnt and Hh proteins are effective *in vitro* and *in vivo* for cell proliferation, hair growth and tissue regeneration. The use of heterogeneous Wnt (e.g., Wnt3a, Wnt 7b, Wnt 10b, etc) and Hh mixtures obtained from characterized normal stem cell cultures is advantageous over the single factors obtained from modified cells engineered to express a particular protein as the combination of a variety of factors is required for a proper stem cell function and therapeutic efficacy.

Thus, disclosed herein are methods for the capture of membrane-bound lipid-modified proteins from stem cells including embryonic stem cells, induced pluripotent stem cells, and adult stem cells. Examples of lipid-modified protein structures include, but are not limited to, proteins (ligands) in the Wnt and the Hh families. The cells are manipulated to maximize the expression of such proteins, then are exposed to cyclodextrins that are known for their ability to capture hydrophobic molecules. The cyclodextrin complexes are further coated with trehalose to confer protection from desiccation and protein denaturation.

Prior to the present disclosure, these lipid-modified proteins were extracted from cells with organic solvents or detergents. These methods have the disadvantage of conferring limited stability and functionality upon the extracted proteins. Organic solvents may denature the protein component and remove the lipid modification that is essential for the protein activity. Detergent extraction results in lipoprotein micelles that can be further included in liposomes. Although the detergent extraction method is superior to solvent extraction, the micelles and liposomes are unstable structures with limited shelf life.

The methods described herein ensure the capture of the lipid-modified proteins and allow the possibility of long term preservation by lyophilization (freeze drying). The cyclodextrin/lipid-modified protein complexes are further preserved using trehalose, a kosmotropic agent that displaces the water surrounding proteins and lipids and ensures structure preservation.

The lipid-modified protein/cyclodextrin complexes isolated from stem cell cultures are useful in tissue repair, wound repair and regeneration, skin rejuvenation, hair growth, and cosmetics.

### Cyclodextrins

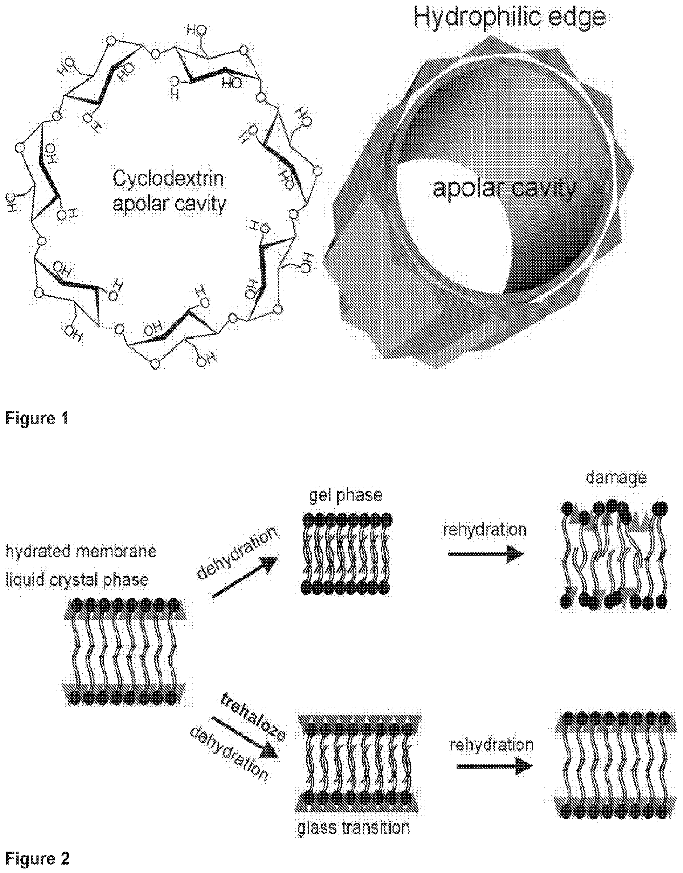

There are three naturally occurring cyclodextrins, -α, -β, and -γ. The cyclodextrins form stable aqueous complexes with many other chemicals. Typical cyclodextrins comprise 6-8 glucopyranoside units, and can be topologically represented as toroids with the larger and the smaller openings of the toroid exposing to the solvent secondary and primary hydroxyl groups respectively. Because of this arrangement, the interior of the toroids is not hydrophobic, but considerably less hydrophilic than the aqueous environment and thus able to host other hydrophobic molecules. In contrast, the exterior is sufficiently hydrophilic to impart the cyclodextrins (or their complexes) with water solubility (Figure 1).

The formation of the inclusion complexes greatly modifies the physical and chemical properties of the guest molecule, mostly in terms of water solubility, thus inclusion complexes of cyclodextrins with hydrophobic molecules are able to penetrate body tissues, and release the biologically active hydrophobic compounds under specific conditions including, but not limited to, pH change, heat, enzymes able to cleave α-1,4 linkages between glucose monomers, or displacement by other hydrophobic molecules (cholesterol for example).

Depending on the number of glucose rings in the molecule, the cyclodextrins are classified as α (alpha)-cyclodextrin (6-membered sugar ring molecule), β (beta)-cyclodextrin (7-membered sugar ring molecule), or γ (gamma)-cyclodextrin (8-membered sugar ring molecule). Because cyclodextrins are hydrophobic inside and hydrophilic outside, they can form complexes with hydrophobic compounds. Thus they can enhance the solubility and bioavailability of such compounds. This is of high interest for pharmaceutical as well as dietary supplement applications in which hydrophobic compounds are delivered. α-, β-, and γ-cydodextrin are all generally recognized as safe by the FDA.

Chemical modifications of the naturally-occurring cyclodextrins can be engineered to increase the solubility, accommodate specific hydrophobic molecules, provide a termination that can be used for attachment to other molecules, provide a specific functionality, such as attachment to specific cell components, and self-assembly in macromolecular structures. Common modifications include random methylation and hydroxypropylation.

Both β-cyclodextrin and methyl-β-cyclodextrin (MBCD) remove cholesterol from cultured cells. The methylated form (MBCD) ismore efficient than β-cyclodextrin at removing cholesterol from cultured cells. The water-soluble MBCD forms soluble inclusion complexes with cholesterol, thereby enhancing its solubility in aqueous solution. MBCD is employed for the preparation of cholesterol-free products; the bulky and hydrophobic cholesterol molecule is easily lodged inside cyclodextrin rings that are then removed. MBCD is also employed in research to disrupt lipid rafts by removing cholesterol from membranes.

### Kosmotropes

Kosmotropes cause water molecules to favorably interact, which also (in effect) stabilizes intramolecular interactions in macromolecules such as proteins. Exemplary kosmotropes include, but are not limited to, propylene glycol, proline, trehalose, ectoine, and trimethylamine N-oxide. Trehalose (mycose, tremalose) is a disaccharide comprised of two glucose molecules.

Trehalose's main biological purpose in mushrooms and bacteria is water regulation, since it forms a gel phase during cellular dehydration protecting organelles during this time and then allows rapid rehydration when a proper environment is reintroduced. It serves a hydration function in humans as well as possessing general antioxidant properties, but its major role is as a cellular chaperone regulating intracellular functions such as protein folding and unfolding.

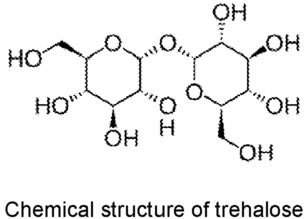

Trehalose has been classified as a kosmotrope or water-structure maker; that is the interaction between trehalose/water is much stronger than water/water interaction and may be involved in its bioprotective action.

Trehalose can inhibit protein aggregation, acting as a stabilizer to improve the shelf-life of therapeutic proteins. Work with model proteins has shown that trehalose is able to abrogate the moisture-induced aggregation of bovine serum albumin by interfering with the formation of intermolecular disulphide bonds. Trehalose is effective in stabilizing lipid membranes and protection against dehydration. The lipid bilayer would otherwise undergo a liquid crystal to gel transition during dehydration, permanently compromising the bilayer structure. Trehalose, by replacing the water, occupies the spaces between lipids and maintains the organized liquid crystal structure upon rehydration (Figure 2).

### Hedgehog (Hh) and Wingless (Wnt) families

Mammals have three Hedgehog homologues, Desert (Dhh), Indian (Ihh), and Sonic (Shh) Hedgehog, of which Sonic is the best studied. The signaling pathways were studied in knockout mice and demonstrated cell specificity for brain, skeleton, musculature, gastrointestinal tract, lungs, and heart. Recent studies point to the role of Hedgehog signaling in regulating adult stem cells involved in maintenance and regeneration of adult tissues. The pathway has also been implicated in the development of some cancers. Drugs that specifically target Hedgehog signaling to fight cancer are being actively developed.

Attachment of lipophilic groups is a widespread modification that occurs on nearly 1,000 proteins of diverse structure and function (Table 1). At least five different types of lipids can be covalently attached to proteins including, but not limited to, fatty acids, isoprenoids, sterols, phospholipids, and glycosylphosphatidyl inositol (GPI) anchors. Proteins can contain more than one type of lipid, e.g. myristate + palmitate, palmitate + cholesterol, or farnesyl + palmitate. The most common outcome of lipid modification is an increased affinity for membranes

**Table 1. Representative lipid-modified proteins**

| **Lipid 1** | **Lipid 2** | **Protein** | **Localization** |
|---|---|---|---|
| Myristate | | Protein kinase A, catalytic subunit | Cytosolic |
| | | MARCKS | Plasma membrane/cytoskeleton |
| | | ARF1 | Golgi<-->cytosol |
| | | c-Src | Plasma membrane/endosomes |
| | Palmitate | Src family kinases (SFKs) | Plasma membrane/endosomes |
| | | Gα subunits | Plasma membrane/cytosol |
| | | AKAPs | Plasma membrane/intracellular organelles |
| Palmitate | | Transferrin Receptor | Plasma membrane |
| | | GPCRs | Plasma membrane |
| | | PSD95 | Postsynaptic density (PSD) |
| | Cholesterol | Hedgehogs (Sonic, Indian, Desert) | Secretory pathway, extracellular space |
| Palmitoleate | | Wnts | Secretory pathway, extracellular space |
| Oleate | | Ghrelin | Secretory pathway, extracellular space |
| Farnesyl | Palmitate | H-Ras, N-Ras | Plasma membrane, Golgi |
| Farnesyl | | K-Ras4B | Plasma membrane |
| | | LaminB | Nuclear envelope |
| Geranylgeranyl | | Rabs, Rhos | Plasma membrane, Golgi, intracellular vesicles |
| Phosphatidyl-ethanolamine | | Atg8/LC3 | Autophagosome |
| GPI anchor | | NCAM | Outer leaflet of plasma membrane |
| | | 5' Nucleotidase | Outer leaflet of plasma membrane |
| | | CD55 | Outer leaflet of plasma membrane |
| | | Thy1 | Outer leaflet of plasma membrane |

The Hh protein is made as a precursor molecule, comprising a C-terminal protease domain and an N-terminal signaling unit, and undergoes a number of unusual modifications during its synthesis. The N terminus of Hh becomes modified by the fatty acid palmitate, on a conserved cysteine residue that is exposed at the very N-terminal end of the protein after its signal sequence has been removed. The palmitoyl group is attached through an amide to the NH₂ group of the cysteine,

Wnt molecules are palmitoylated and are therefore much more hydrophobic than predicted from their primary amino acid sequences. The amino acid of Wnt proteins that appears to be modified is the first conserved cysteine (C77), a residue that is present in all Wnts and that is essential for Wnt function, as revealed by mutant analysis.

Because lipid modification which confers hydrophobicity, Hh and Wnt cannot be distributed systemically; the proteins are membrane-bound and can only be transmitted from cell to cell amongst cells that are in direct contact. In contrast, soluble factors (such as FGF, EGF etc) are distributed systemically and can exercise effects on regional or distant cells.

An originating cell (stem cell) expressing the Engrailed (En) transcription factor secretes Hh. Only cells adjacent to En-expressing cells are able to respond to Hedgehog following interaction of Hh with the receptor protein Patched (Ptc).

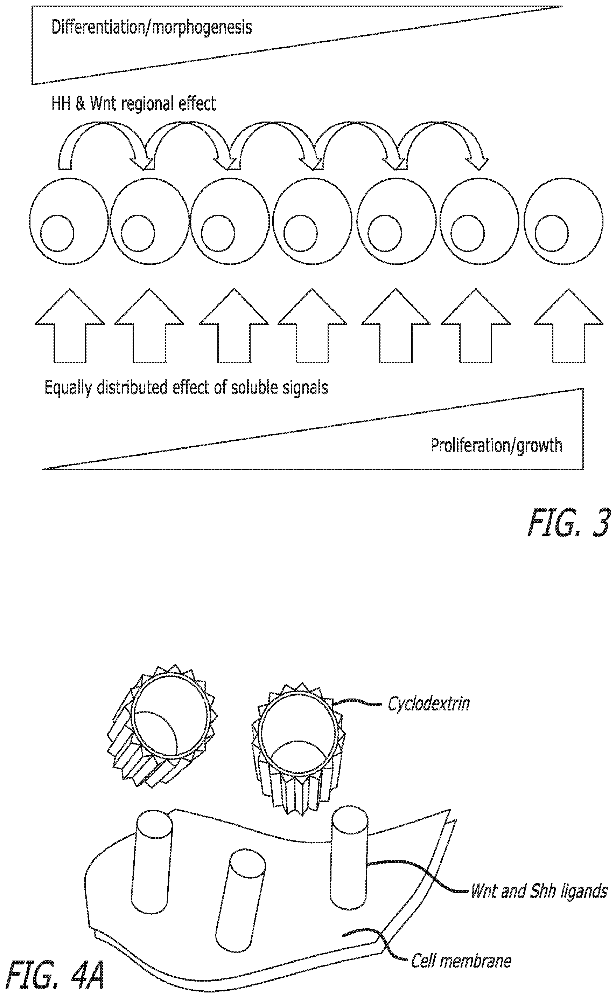

Cells with Hh-activated Ptc synthesize the Wnt protein. The Wnt lipid-modified protein acts as an intercellular signal and patterns the adjacent rows of cells by activating its cell surface receptor Frizzled. Thus, the effects of Wnt and Hh on adjacent cells establishes a positional code that accounts for the distinct anatomical features, while the soluble factors establish a temporary code for cell proliferation and tissue growth (Figure 3).

The hair follicle is considered a mini-organ formed with neuroectodermal-mesodermal interaction. Hair follicle neogenesis occurs in the embryo by invagination of the epidermal placode into the surrounding dermis. Postnatal follicles undergo a cycle of renewal in 3 phases: anagen (growth), catagen (regression), and telogen (resting). The first complete postnatal hair folicle cycle (first anagen, first catagen, first telogen) is completed in the first 3.5 weeks after birth and is followed by the second hair cycle (second anagen, second catagen, second telogen).

In skin, the formation of hair follicles from developing epidermis requires signals from fibroblasts in the underlying dermis. Hair follicle morphogenesis takes place during the late embryonic and early neonatal period. Adult skin does not normally give rise to new follicles.

Hair follicle neogenesis can be induced in adult mouse skin in response to transgenic or wound-induced epidermal activation of Wnt/β-catenin. Inhibition of Wnt signaling by DKK1 (Dickkopf-related protein 1) demonstrates the functional importance of Wnt signaling in hair follicle development. Several Wnt molecules are expressed in the hair follicle and could serve this function. Wnt3a and Wnt7a are expressed in the follicular matrix cells and maintain dermal papilla cells in the anagen phase. These cells are likely to be capable of responding to Wnt because they express components of the Wnt signal transduction cascade including frizzled7, disheveled2, GSK3β, β-catenin, and Lef1. Thus, the Wnt pathway is considered to be the master regulator during hair follicle morphogenesis. Wnt signaling proceeds through EDA/EDAR/NF-κB (ectodysplasin A/ectodysplasin A receptor/ nuclear factor kappa-light-chain-enhancer of activated B cells) signaling. NF-κB regulates the Wnt pathway and acts as a signal mediator by upregulating the expression of Shh. Dermal Shh and platelet-derived growth factor (PDGF) signaling up-regulates dermal noggin expression; noggin is a potent inhibitor of bone morphogenic protein (BMP) signaling which helps in counteracting BMP-mediated β-catenin inhibition. This interplay of signaling between the epithelial and dermal lineage helps in epithelial Shh signal amplification.

The relevance of Shh to hair development has been suggested by the Shh expression pattern during embryogenesis and by manipulation of Shh expression throughout embryonic development. During normal hair follicle development, Shh is expressed in follicles in the epidermal placode, and its receptor Ptc is detected in underlying mesenchymal condensation at an early embryonic age.

*In vivo* experiments have suggested that Shh stimulates the transition from telogen to anagen possibly in collaboration with other local factors. Transient expression of Shh could re-activate the hair growth cycle in disease conditions.

In mammals, despite considerable ability for tissue regeneration, large wounds result in the formation of scar tissue instead of a complete restoration of tissue morphology and function. This limited regenerative capacity is partly due to rapid interposition of fibrotic tissue, something that prevents subsequent tissue regeneration, but might be a defensive advantage in preventing harmful microbes. If injured, only bone, liver, and infant finger tips can regenerate. Aging is another determinant for tissue restoration, as animals gradually lose their regenerative capacity as they get older.

Repaired skin, which usually heals as a scar, is weaker than intact skin, and contains a disorganized extracellular matrix (ECM) compared to non-wounded skin. Cutaneous wounds do not normally regenerate hair follicles. As a result, postnatal mammalian skin repair is not identical to the process of regeneration of early gestational fetal wounds in which the regenerated tissue is almost indistinguishable from the uninjured tissue.

Wnt proteins may participate in stimulating dermal β-catenin during wound repair, although Wnt signaling is not crucial for maintaining elevated β-catenin levels during the proliferative phase of cutaneous healing. Analogous to its function in skin development, Wnt and/or β-catenin signaling plays an important role in various aspects of cutaneous wound repair, involved in the construction of epithelial structures and in the reconstitution of the dermal compartment.

The relative level and activity of β-catenin contributes to the dermal wound phenotype, with high β-catenin levels and activity leading to an enlarged, hypercellular dermal compartment, thus contributing to a hypertrophic scar formation. β-catenin levels are Wnt-independent, likely influenced by the transforming growth factor beta (TGFβ) levels.

Wnt signaling regulates cell proliferation in the adult epidermis, which directly impacts the rate and extent of skin wound healing. Wnts also serve as niche signals for at least two types of skin stem cells, those in the bulge region of the hair follicle and those in the basal layer of the interfollicular epidermis, and these stem cells contribute to cutaneous wound repair. Topical application of liposomal Wnt3a to a non-healing wound supplements endogenous Wnt signaling, and results in better skin wound healing.

Hedgehog signaling was shown to directly contribute for normal and accelerated wound healing in mice. When Hh signaling is inhibited, all aspects of wound healing (wound closure, epithelialization, granulation formation, vascularity, and proliferation) are severely impaired.

In the skin, touch domes develop in tandem with primary hair follicles and contain sensory Merkel cells. Dermal Wnt signaling, and subsequent epidermal Eda/Edar (ectodysplasin/ectodysplasin receptor) signaling, promote Merkel cell morphogenesis by inducing Shh expression in early follicles. Although developmentally associated with hair follicles, fate mapping demonstrated Merkel cells primarily originated outside the hair follicle lineage. These findings suggest that touch dome development requires Wnt-dependent mesenchymal signals to establish reciprocal signaling within the developing ectoderm Shh signaling from primary follicles to extrafollicular Merkel cell progenitors. Locally-produced Shh acting as a morphogen is essential for lineage specification during development and postnatal touch dome stem cell maintenance.

In amphibians, Hh signaling, and its hierarchical correlation with respect to Wnt signaling, controls limb regeneration. Wnt signaling has been shown to promote self-renewal in both gut epithelial and hematopoietic stem cells (HSCs). Stem cells in many tissues are responsive to Wnt (Table 2).

**Table 2. Examples of Wnt-responsive tissue stem cells identified by means of lineage tracing.**

| **Tissue** | **Stem cell** |
|---|---|
| Intestine | Crypt base columnar cell |
| Mammary gland | Basal cell |
| Stomach | Basal pyloric cell |
| Interfollicular epidermis | Basal cell |
| Central nervous system | Radial glial cell |
| Hair follicle | Outer bulge cell |
| Kidney | Nephron segment-specific stem cell |
| Cochlea | Tympanic border |
| Ovary | Hilum ovarian surface epithelial cell |
| Taste bud | Circumvallate papilla stem cell in posterior tongue |
| Retina | Retinal progenitor cells |

Along with the lipid-modified proteins bound to cell membranes, lipids are also involved in cell signaling including, but not limited to, sphingolipid based lipids (*e*.*g*., ceramide, sphingosine, sphingosine-1-phosphate, glucosylceramide, ceramide-1-phosphate, phosphatidylinositol bisphosphate (PIP₂) lipid agonist; phosphatidylinositol based lipids (*e*.*g*., phosphatidylinositol bisphosphate (PIP2)); activators of G-protein coupled receptors (*e*.*g*., lysophosphatidic acid (LPA), sphingosine-1-phosphate (S1P), platelet activating factor (PAF), endocannabinoids, prostaglandins, FAHFA, retinol derivatives); and activators of nuclear receptors (*e*.*g*., steroid hormones, retinoic acid, prostaglandins).

### Compositions

Thus, disclosed herein are compositions comprising lipid-modified Hedgehog (Hh) and/or Wingless (Wnt) proteins and at least one cyclodextrin. The lipid-modified Hh and/or Wnt proteins are isolated from human stem cells as described herein. In certain embodiments, the stem cells are pluripotent, multipotent, single lineage dividing progenitors, or immortalized cell lines.

In certain embodiments, the source cells for the Wnt and Hh proteins are human embryonic, parthenogenic, or induced pluripotent stem cells. Other cells of interest include any *in vitro* proliferating cells that have been identified as fetal or adult stem cells, or sourced from fetal annexes. Other cells can be modified with a genetic manipulation that confers immortality by cell cycle deregulation, for example telomerase expression. In certain embodiments, the stem cells are immortalized by the genetically engineered expression of telomerase reverse transcriptase (hTERT). Source cells can be cultivated using established methods and cell culture media as known to persons of ordinary skill in the art.

In some embodiments, the Hh or Wnt proteins are harvested by: first discarding the culture media, rinsing the cultures with an isotonic buffer (*e.g*. saline, Hanks, balanced salt solution etc), then combining cells with of a harvesting solution for about 1 hour to about 5 hours with slow, continuous, or intermittent agitation. Some of the cells may lose the attachment to substrate. The harvested solution, containing the soluble lipid-modified protein/cyclodextrin complexes is further filtered to remove debris (example through a 0.1-0.5 µm) filter and stored at 4°C.

Harvesting solutions suitable for obtaining lipid-modified Wnt and Hh proteins from stem cells comprise isotonic solutions containing about 1-20 mM of a cyclodextrin. In some embodiments, the concentration of cyclodextrin is about 1-5 mM, about 5-10 mM, about 10-15 mM, about 15-20 mM, about 2-10 mM, about 5-20 mM, about 8-20 mM, about 12-20 mM, about 8-12 mM, about 5 mM, about 7 mM, about 9 mM, about 10 mM, about 11 mM, about 13 mM, or about 15 mM. The volume of harvesting solution is about 0.1-1.0 mL/cm² of dish or flask. In some embodiments, the volume of harvesting solution is about 0.25 mL/cm² of dish or flask.

The harvesting solution is incubated with the source cells for about 1 hour to about 5 hours with slow, continuous, or intermittent agitation. In some embodiments, the harvesting solution is incubated with the cells for about 1 hour, about 1.5 hours, about 2 hours, about 2.5 hours, about 3 hours, about 3.5 hours, about 4 hours, about 4.5 hours, or about 5 hours.

In some embodiments, the cyclodextrin is one or more of an α-cyclodextrin, a β-cyclodextrin, or a γ-cyclodextrin. In some embodiments, the, natural cyclodextrins are chemically modified by hydrogenation, hydroformylation, oxidation, reduction and carbon-carbon coupling reactions. Such well known modifications include 2-hydroxypropyl β-cyclodextrin and methyl-β-cyclodextrin. In some embodiments, the cyclodextrin is methyl-β-cyclodextrin (MBCD).

In certain embodiments, the harvest solution further comprises a kospmotrope. One exemplary method for harvest solution preparation includes the addition of a kosmotropic agent that displaces the water surrounding the protein molecule. An exemplary kosmotropic agent is trehalose that is added in the cyclodextrin complex solution for a final concentration between about 5% and about 30%. In other embodiments, the kosmotrope concentration is about 5% to about 10%, about 10% to about 15%, about 15% to about 20%, about 15% to about 25%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, or about 30%, or any concentration bounded by these values. In one embodiment, the trehalose concentration is 20% w/v added immediately after harvesting.

In some embodiments, the harvest solution comprises 10 mM cyclodextrin and 20% trehalose in water.

The cyclodextrin complex can be further preserved by lyophilization using a low temperature method (*e.g*., freeze-drying).

The detection of Wnt and Hh proteins in the disclosed compositions can be accomplished using a commercially available quantitative ELISA detection kits.

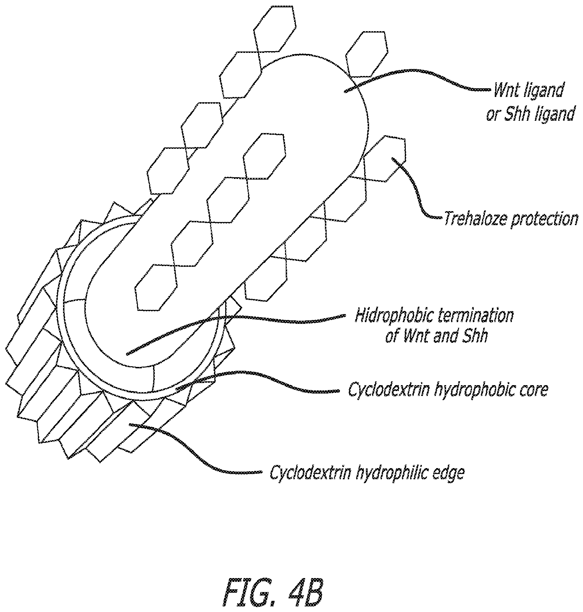

One embodiment of the lipid-modified protein/cyclodextrin complexes disclosed herein is depicted in Figures 4A and 4B.

The use of cyclodextrin Wnt/Hh complexes can be formulated for *in vivo* applications observing the following principles: (1) preparation and storage temperature should be maintained below 40°C; (2) the final product should not contain hydrophobic molecules that could displace the cyclodextrin payload (that is, the lipid-modified proteins); and (3) can include an antimicrobial stabilizing agent or preservative such as, but not limited to, silver (Ag) nano- or micro-particles, phenoxyethanol, caprylyl glycol, penthylene glycol or other agents used for topical applications.

In one exemplary embodiment, a composition comprising Wnt/Hh-cyclodextrin complexes is an aqueous solution, that optionally further includes silver particles. The composition can further include one or more of amino acids, peptides, proteins, hydrosoluble vitamins and microelements.. Exemplary components of the compositions include, but are not limited to hydro-soluble growth factors and steroid hormones and analogs thereof. Exemplary hydro-soluble growth factors include, but are not limited to, fibroblast growth factor (FGF), epidermal growth factor (EGF), keratinocyte growth factor (KGF), hepatocyte growth factor (HGF), etc.

Silver particles (nanoparticles or microparticles) can be included at a concentration of 0.01-1.0 % w/v. In some embodiments, the silver particles are included at a concentration of 0.1-0.55 w/v.

According to some embodiments, a topical composition or formulation prepared according to the present disclosure may take the compositional form of a liquid, a paste, a cream, a lotion, a powder, an ointment, or a gel.

According to some embodiments, the compositional form is a paste, meaning a semisolid dosage form that contains one or more substances intended for topical application.

According to some embodiments, the compositional form is a cream. The term "cream" as used herein refers to a viscous liquid or semisolid emulsion of either the oil-in-water or water-in-oil type. As used herein "emulsion" refers to a colloid system in which both the dispersed phase and the dispersion medium are immiscible liquids where the dispersed liquid is distributed in small globules throughout the body of the dispersion medium liquid. A stable basic emulsion contains at least the two liquids and an emulsifying agent. Common types of emulsions are oil-in-water, where oil is the dispersed liquid and an aqueous solution, such as water, is the dispersion medium, and water-in-oil, where, conversely, an aqueous solution is the dispersed phase. It also is possible to prepare emulsions that are nonaqueous. Creams of the oil-in-water type include hand creams and foundation creams. Water-in-oil creams include cold creams and emollient creams.

According to some embodiments, the compositional form is a lotion, meaning a liquid or semi-liquid preparation that contains one or more active ingredients in an appropriate vehicle. A lotion may be a suspension of solids in an aqueous medium, an emulsion, or a solution.

A "solution" generally is considered as a homogeneous mixture of two or more substances. It is frequently, though not necessarily, a liquid. In a solution, the molecules of the solute (or dissolved substance) are uniformly distributed among those of the solvent. Solvents that may be useful in the compositions of the present disclosure include water, as well as organic solvents, such as the alcohols.

According to some embodiments, the compositional form is an ointment. An ointment is a semi-solid preparation often intended for external application to the skin. Generally, ointment bases are categorized into hydrocarbon bases (oleaginous), adsorption bases (anhydrous); emulsion bases (water and oil type); and water soluble bases. Due to their anhydrous nature, ointments generally do not require any preservatives. They are more moisturizing and more occlusive than creams, and form a protective film over the skin. The occlusive effect tends to prolong and enhance penetration.

According to some embodiments, the compositional form of the present disclosure is a gel. The term "gel" as used herein refers to a sticky, jelly-like semisolid or solid prepared from high molecular weight polymers in an aqueous or alcoholic base.

Additional compositional forms may be prepared using technology readily known in the formulation arts, such as those described in Remington: The Science and Practice of Pharmacy, 20th Ed. (Gennaro, A.R. et al., eds) Lippincott Williams & Wilkins: Philadelphia (2000), which is incorporated herein by reference.

A number of additional ingredients can be added to the compositions disclosed herein for functional, esthetic, and marketing purposes, including emulsifying agents, preservatives, humectants, thickeners, fragrances, dyes, herbal extracts, and vitamins, provided that the selected additional component(s) is chemically and physically compatible. The term "compatible" is used herein to mean that the components of the compositions are capable of being combined with each other in a manner such that there is no interaction that would substantially reduce the efficacy of the compositions under ordinary use conditions.

According to some embodiments, the compositions comprise a polysorbate, e.g., polysorbate- 20, polysorbate-40, polysorbate-80, and mixtures thereof.

The term "carrier" as used herein refers to a pharmaceutically acceptable inert agent or vehicle for delivering one or more active agents to a subject, and often is referred to as "excipient." The carrier must be of sufficiently high purity and of sufficiently low toxicity to render it suitable for administration to the subject being treated. The carrier further should maintain the stability and bioavailability of lipid-modified protein/cyclodextrin complexes disclosed herein. The carrier can be liquid or solid and is selected, with the planned manner of administration in mind, to provide for the desired bulk, consistency, etc., when combined with an active agent and other components of a given composition.

According to some embodiments, the described compositions comprise an aqueous carrier. The level and species of the carrier are selected according to the compatibility with other components, and other desired characteristic of the product. The aqueous carrier is contained in the compositions at a level by weight of, for example, about 30% to about 98%, about 50% to about 95%, or about 70% to about 95%.

Exemplary carriers include water and water solutions of lower alkyl alcohols. Exemplary lower alkyl alcohols include monohydric alcohols having 1 to 6 carbons, e.g., ethanol. According to some embodiments, the aqueous carrier is substantially water.

The pH of the described compositions are, for example, about 4 to about 8 When skin benefiting agents are included in the compositions, the pH may be adjusted to that which provides optimum efficacy. Buffers and other pH adjusting agents can be included to achieve the desirable pH. Exemplary pH adjusters herein include acetates, phosphates, citrates, triethanolamines and carbonates.

The viscosity (resistance to flow) of the described compositions may vary over a wide range, and may depend on viscosifying agents. For example, according to some embodiments, the described compositions may comprise a viscosifying agent that provides the compositions with a viscosity of from about 500 mPas to about 1,000,000 Pas. According to some embodiments, the viscosifying agent provides the compositions with a viscosisty of about 1,000 mPas to about 100,000 mPas.

Carboxylic acid/carboxylate copolymers are nonlimiting examples of viscosifying agents used for providing microemulsions. Such copolymers can keep the composition at a suitable viscosity without being tacky or greasy upon use, and can disperse and stabilize water insoluble components of the composition when such components are included. Exemplary commercially available carboxylic acid/carboxylate copolymers include acrylates/C₁₀₋₃₀ alkyl acrylate crosspolymers, *e.g.*, PEMULEN^{™} TR-I, PEMULEN^{™} TR-2, CARBOPOL^{®} 1342, CARBOPOL^{®} 1382, and CARBOPOL^{®} ETD 2020, all available from B. F. Goodrich Company.

Neutralizing agents, *e.g*., sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof, may be included to neutralize the carboxylic acid/carboxylate copolymers.

Exemplary cellulose derivative polymers include, without limitation, methylcellulose, ethylcellulose, hydroxyethylcellulose, hydroxyethyl ethylcellulose, hydroxypropyl methyl cellulose, nitrocellulose, sodium cellulose sulfate, sodium carboxymethylcellulose, crystalline cellulose, cellulose powder, and mixtures thereof. According to some embodiments, the cellulose derivative polymers are hydroxyethylcellulose, carboxymethylcellulose, and mixtures thereof. Commercially available compounds that are highly useful herein include hydroxyethylcellulose with tradename Natrosol Hydroxyethylcellulose, and carboxymethylcellulose with tradename Aqualon Cellulose Gum, both available from Aqualon.

Other exemplary viscosifying agents include pullulan, mannan, scleroglucans, polyvinylpyrrolidone, polyvinyl alcohol, guar gum, hydroxypropyl guar gum, xanthan gum, acacia gum, arabia gum, tragacanth, galactan, carob gum, karaya gum, locust bean gum, carrageenin, pectin, amylopectin, agar, quince seed *(Cydonia oblonga* Mill), starch (rice, corn, potato, wheat), and algae colloids (algae extract). Exemplary microbiological polymers include, without limitation, dextran, succinoglucan, starch-based polymers such as carboxymethyl starch, and methylhydroxypropyl starch. Exemplary alginic acid-based polymers include, without limitation, sodium alginate, and alginic acid propylene glycol esters. Exemplary acrylate polymers include, without limitation, sodium polyacrylate, polyacrylamide, and polyethyleneimine. Exemplary inorganic water soluble material includes, without limitation, bentonite, aluminum magnesium silicate, laponite, hectonite, and anhydrous silicic acid.

Polyalkylene glycols having a molecular weight of more than about 1000 also are exemplary viscosifying gents. Exemplary compounds include polyethylene oxides, polyoxyethylenes, and polyethylene glycols, polypropylene oxides, polyoxypropylenes, and polypropylene glycols; and polypropylene glycols and mixed polyethylene- polypropylene glycols, or polyoxyethylene- polyoxypropylene copolymers. Exemplary polyethylene glycol polymers include, without limitation, PEG-2M, also known as POLYOX WSR^{®} N-10, which is available from Union Carbide and available as PEG-2,000); PEG-5M, also known as POLYOX WSR^{®} N-35; and POLYOX WSR^{®} N-80, both available from Union Carbide and as PEG-5,000 and Polyethylene Glycol 300,000); PEG-7M, also known as POLYOX WSR^{®} N-750 (available from Union Carbide); PEG-9M, also known as POLYOX WSR^{®} N-3333 (available from Union Carbide); and PEG-14 M, also known as POLYOX WSR^{®} N-3000 available from Union Carbide).

Exemplary commercially available additional water soluble polymers include, without limitation, xanthan gum (KELTROL^{™}, available from Kelco), Carbomers (CARBOPOL^{™} 934, CARBOPOL^{™} 940, CARBOPOL^{™} 950, CARBOPOL^{™} 980, and CARBOPOL^{™} 981(all available from B. F. Goodrich Company), acrylates/steareth-20 methacrylate copolymer (ACRYSOL^{™} 22 (available from Rohm and Hass), polyacrylamide (SEPIGEL^{™} 305 (available from Seppic), glyceryl polymethacrylate (LUBRAGEL^{™} NP, and a mixture of glyceryl polymethacrylate, propylene glycol and PVM/MA copolymer (LUBRAGEL^{™} OIL (available from ISP), scleroglucan (CLEAROGEL^{™} SCI I available from Michel Mercier Products Inc. (NJ, USA)), ethylene oxide and/or propylene oxide based polymers (CARBOWAX^{™} PEGs, POLYOX^{™} WASRs, and UCON^{™} FLUIDS (all supplied by Amerchol).

Other exemplary agents include commercially available amphoteric polymers such as Polyquaternium 22 (MERQUAT^{™} 280, MERQUAT^{™} 295), Polyquaternium 39 (MERQUAT^{™} PLUS 3330, MERQUAT^{™} PLUS 3331), and Polyquaternium 47 (MERQUAT^{™} 2001, MERQUAT^{™} 200 IN), all available from Calgon Corporation.

The term "humectants" as used herein refers to substances that promote water retention due to their hygroscopicity. They act by being absorbed into the skin and attract water from the atmosphere. The attracted water then serves as a reservoir for the stratum corneum.

Exemplary water-soluble humectants include, without limitation, polyhydric alcohols, such as butylene glycol (1,3 butanediol), pentylene glycol (1,2-pentanediol), glycerin, sorbitol, propylene glycol, hexylene glycol, ethoxylated glucose, 1,2-hexane diol, 1,2-pentane diol, hexanetriol, dipropylene glycol, erythritol, trehalose, diglycerin, xylitol, maltitol, maltose, glucose, fructose; and other water-soluble compounds such as urea, sodium chondroitin sulfate, sodium hyaluronate, sodium adenosin phosphate, sodium lactate, pyrrolidone carbonate, glucosamine, cyclodextrin, and mixtures thereof. Additional examples include water soluble alkoxylated nonionic polymers such as polyethylene glycols and polypropylene glycols of molecular weight of up to about 1000 (e.g., PEG-200, PEG-400, PEG-600, PEG-1000), and mixtures thereof.

Commercially available humectants include, without limitation: butylene glycol (1,3-Butylene glycol, available from Celanese), pentylene glycol (HYDROLITE^{™}-5 available from Dragoco), glycerin (STAR^{™} and SUPEROL^{™}, available from The Procter & Gamble Company, CRODEROL^{™} GA7000 available from Croda Universal Ltd., PRECERIN^{™} series available from Unichema, and a same tradename as the chemical name available from NOF; propylene glycol (LEXOL^{™} PG- 865/855 available from Inolex, 1,2-PROPYLENE GLYCOL USP available from BASF; sorbitol (LIPONIC^{™} series available from Lipo, SORBO^{™}, ALEX^{™}, A-625^{™}, and A-641^{™} available from ICI, and UNISWEET^{™} 70, UNISWEET^{™} CONC available from UPI; dipropylene glycol with the same tradename available from BASF; diglycerin (DIGLYCEROL^{™}, available from Solvay GmbH); xylitol with the same tradename available from Kyowa and Eizai; maltitol (MALBIT^{™} available from Hayashibara; sodium chondroitin sulfate with the same tradename available from Freeman and Bioiberica, and with tradename ATOMERGIC SODIUM CHONDROITIN SULFATE available from Atomergic Chemetals; sodium hyaluronate, available from Chisso Corp. the same with tradenames ACTIMOIST^{™} available from Active Organics, AVIAN SODIUM HYALURONATE series, available from Intergen, HYALURONIC ACID Na, available from Ichimaru Pharcos; sodium adenosine phophate with the same tradename available from Asahikasei, Kyowa, and Daiichi Seiyaku; sodium lactate with the same tradename available from Merck, Wako, and Showa Kako, cyclodextrin (CAVITRON^{™} available from American Maize, RHODOCAP^{™} series available from Rhone-Poulenc, and DEXPEARL^{™} available from Tomen); polyethylene glycols (CARBOWAX^{™} series available from Union Carbide), and a mixture of glyceryl polymethacrylate, propylene glycol and PVM/MA copolymer (LUBRAJEL^{™} Oil available from Guardian Lab).

The term "preservative" is used herein to refer to substances that prevent or inhibit the growth of undesired microorganisms in products that contain water. Preservatives approved for use in pharmaceuticals, such as topical formulations, may be identified in the current Federal Regulations published in volume 21 of the Code of Federal Regulations, which is incorporated herein by reference. Exemplary preservatives include, without limitation: ascorbic acid, ascorbyl palmitate, biopein, BHT (butylated hydroxyl-toluene), butylated hydroxyanisole, butylated hydroxytoluene, butylparaben, calcium ascorbate, calcium sorbate, citric acid, cinnamon cassia, chlorocresol, diazolidinyl urea, dilauryl thiodipropionate, EDTA (ethylenediamine tetraacetic acid tetrasodium salt), erythorbic acid, grapefruit seed extract, hydroxyhenzoates, methylparaben, Neopein, phenonip, phenoxyethanol, potassium bisulfite, potassium metabisulfite, potassium sorbate, propylparaben, rosemary oil extract, sodium ascorbate, sodium benzoate, sodium bisulfite, sodium metabisulfite, sodium sorbate, sodium sulfite, sorbic acid, sulfur dioxide, Suprarein, thiodipropionic acid, silver particles, and/or tocopherols. Additionally, preservation may also be accomplished by storage at reduced temperatures (e.g., below 4°C, or frozen).

In some embodiments, the compositions are applied topically once daily, twice daily, three times daily, every other day, weekly, or for any time period necessary to achieve the desired results. Typically the compositions are applied topically to the desired treatment area and allowed to absorb into the skin

### Therapeutic Uses

Therapeutic uses for the cyclodextrin/lipid-modified protein complex-containing compositions include but are not limited to, hair growth or regrowth purposes, the *in vitro* expansion of hair follicle stem cells for the generation of hair follicles that can be transplanted in the autologous recipient, wound healing, restoration of tactile sensation, improvement of the appearance of skin, and tissue rejuvenation.

The tissue targeted by the cyclodextrin complexes should contain a receptor for the payload protein (e.g., Frizzled, Patched, etc.) and an exchange hydrophobic molecule, for example cholesterol from a cell membrane or a lipidic secretion, for example sebum.

The compositions and formulations disclosed herein can be administered topically. Cyclodextrin complexes-containing compositions can be topically administrated in doses containing about 0.1% to about 100% of the harvesting solution. In certain embodiments, the compositions are administered topically at a dose of about 5% to about 25% (v/w, v/v or w/v), about 10% to about 25%, about 15% to about 25%, about 20% to about 25%, about 5% to about 20%, about 5% to about 15%, about 5% to about 10%, about 5%, about 7.5%, about 10%, about 12.5%, about 15%, about 17.5%, about 20%, about 22.5%, or about 25% or any range bounded by these values.

Dosages and desired drug concentrations of compositions disclosed herein may vary depending on the particular use envisioned. The determination of the appropriate dosage is well within the skill of an ordinary physician. Animal experiments provide reliable guidance for the determination of effective doses for human therapy. Interspecies scaling of effective doses can be performed following the principles laid down by Mardenti, J. and Chappell, W. "The use of interspecies scaling in toxicokinetics" In Toxicokinetics and New Drug Development, Yacobi et al, Eds., Pergamon Press, New York 1989, pp. 42-96. The term "therapeutically effective" amount as used herein refers to the amount needed to perform the particular treatment such as, for example, hair growth. "Treatment" refers to both therapeutic treatment and prophylactic or preventative measures, wherein the object is to prevent or slow down (lessen) the targeted condition or disorder. Those in need of treatment include those already with the disorder as well as those prone to have the disorder or those in whom the disorder is to be prevented. In some embodiments, the disorder is present.

For use in hair growth or re-growth, a composition disclosed herein can be applied on a healthy scalp to maintain an extended anagen or in various forms of alopecia to convert from telogen to anagen or to induce *de novo* hair growth. In certain embodiments, the compositions disclosed herein are applied topically to a portion of the scalp to induce hair growth or hair re-growth. In other embodiments, the compositions are applied to other areas of the body where hair growth or hair regrowth is desired such as, but not limited to, eyebrows, eyelashes, face, chest, arms, legs, or genital area.

In certain embodiments, a composition disclosed herein is applied topically for wound healing, such as for accelerated healing of atonic wounds or scarless wound healing.

In other embodiments, the compositions disclosed herein are applied topically to wounds to aid in wound healing.

In yet other embodiments, the compositions disclosed herein are applied topically to existing scars to minimize the appearance of the scar.

In yet other embodiments, the compositions disclosed herein are applied topically to areas of decreased tactile sensation to improve tactile sensation.

In some embodiments, the compositions disclosed herein are applied topically to the skin to improve youthful appearance, reduce wrinkles, improve skin elasticity, counteract the visible effects of skin aging and environmental stresses, reduce hyperpigmentation or hypopigmentation, improve the appearance of age spots, improve skin texture, and/or improve homogeneity of skin pigmentation.

### EXAMPLES

### Example 1. Capture and detection of HhIWnt from cells derived from partially differentiated embryonic stem cell cultures.

Embryonic stem cells were expanded according to current published methods using a serum free media supplemented with bFGF (10 ng/mL) and activin A (5 ng/mL) on an adherent substrate consisting on a thin layer of MATRIGEL^{®}. After confluence, half of the cultures were fed with the same media not including the growth factors bFGF and Activin A. A cell culture supernatant sample was analyzed for follistatin concentration.

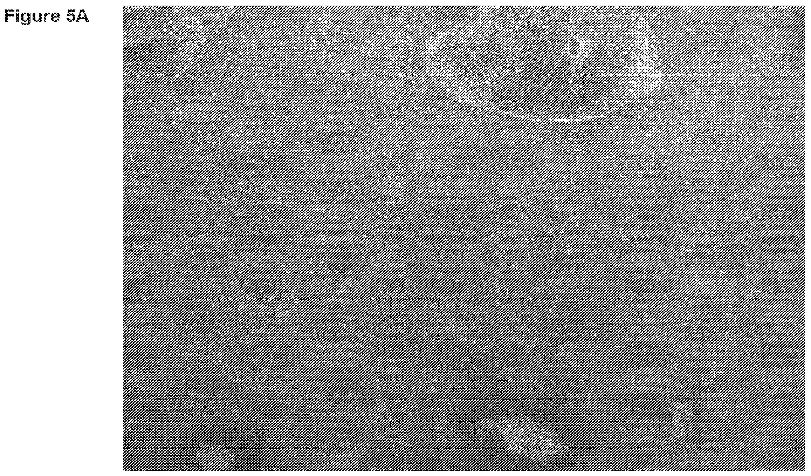

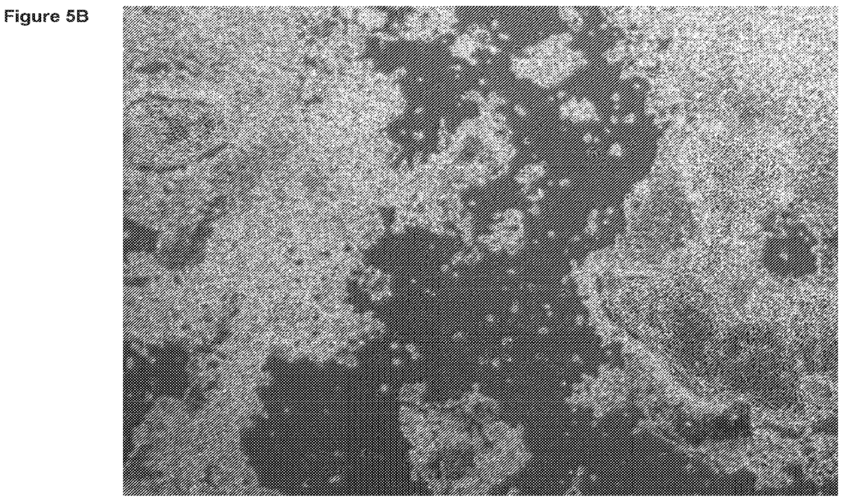

Individual cultures, undifferentiated or partial differentiated, were exposed to 10 mM solution of α-cyclodextrin, β-cyclodextrin, γ-cyclodextrin, or methyl-β-cyclodextrin (MBCD) for 60 min to 180 min. The cell cultures before exposure were smooth, compact, and multilayered (Figure 5A). After incubation with a cyclodextrin, the cultures were disrupted with the majority of cells losing adherence (Figure 5B).

The MBCD solution incubated with the cells was then analyzed for the concentration of Shh, Wnt 3a, and Wnt7a. The results are presented in Table 3.

**Table 3. Concentration of follistatin in the supernatant media of the cell culture prior exposure to cyclodextrins and the concentration of Wnt and Shh in the methyl-betacyclodextrin capture solution after 3 hr exposure to the cell culture.**

| | Undifferentiated hESC (ng/mL) | Partial differentiated hESC (ng/mL) | Media control |
|---|---|---|---|
| Follistatin (in supernatant) | 0.830 | 9.562 | 0 |
| Wnt3a | 7.950 | 7.720 | 0 |
| Wnt7b | 27.026 | 24.658 | 0 |
| Shh | 0.000 | 570.900 | 0 |

Addition of α- or β-cyclodextrin demonstrated a reduced but proportional effect.

### Example 2. Comparison study for hair growth in an animal model

A culture of partial-differentiated embryonic stem cells was exposed to a harvest solution containing 10 mM methyl-β-cyclodextrin and 20% trehalose in water for injection, at a volume of 1 mL/10⁶ cells for 3 hours at room temperature to obtain the cyclodextrin/lipid-modified protein complexes subsequently referred as "active ingredient".

The formulated active ingredient (containing phenoxyethanol and caprylyl glycol 0.75% as preservative) was tested targeting hair growth (or re-growth). A mouse model for hair growth was used to test a prototype formulation. Male and female mice 6 weeks of age in confirmed first telogen phase were randomized in the treatment groups for 3 different concentrations and a negative control.

On Day -1, all the animals were anesthetized with isoflurane and the entire back (from shoulders to haunches) was freed of hair by clippers. The test articles were applied topically for 14 days continuously starting on Day 0; test articles were rubbed gently into the dorsal skin of each mouse daily. New gloves were worn for each treatment type. Animals were single housed during treatment period to avoid cage-mates licking the test article.

Body weights and clinical observations were measured weekly. Macroscopic digital photographs were taken on Days 2, 7, 10, 14, and 21. On Day 21, skin samples from all mice were collected and fixed in formalin for histology analysis.

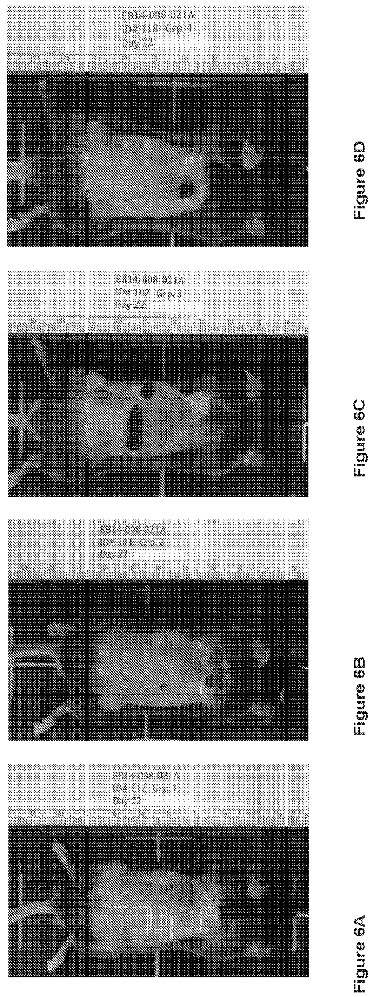

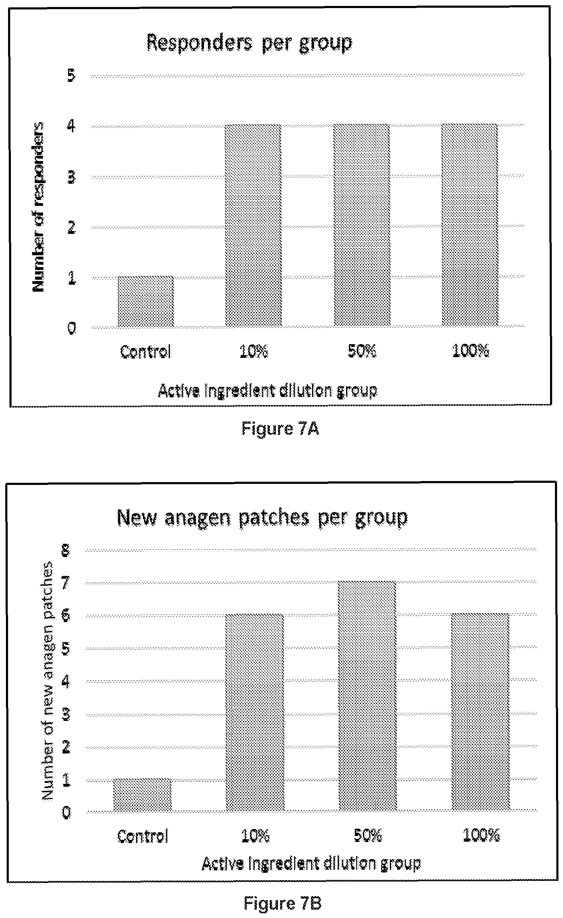

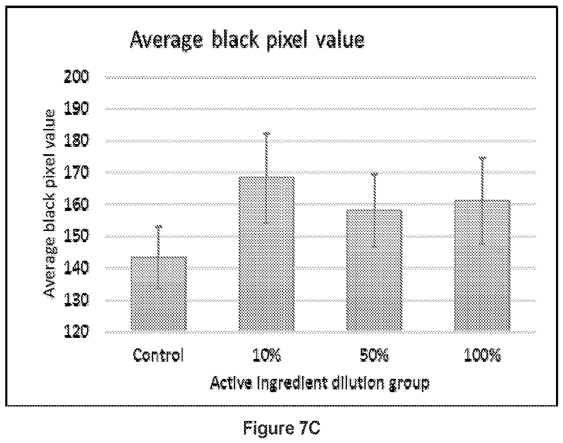

All treated groups responded by new anagen patches regardless of concentration of active ingredient. The response confirmed by increased darkness of the skin and the count of new anagen patches in the shaved area (Figures 6, 7A-C)

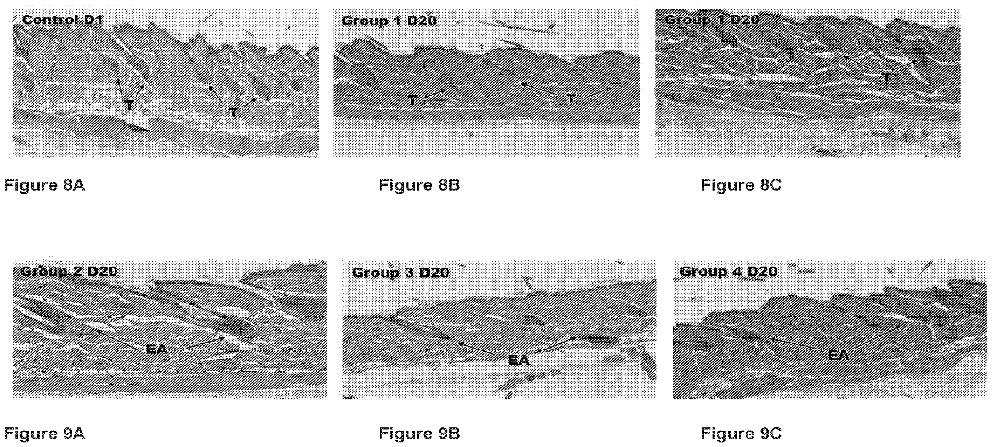

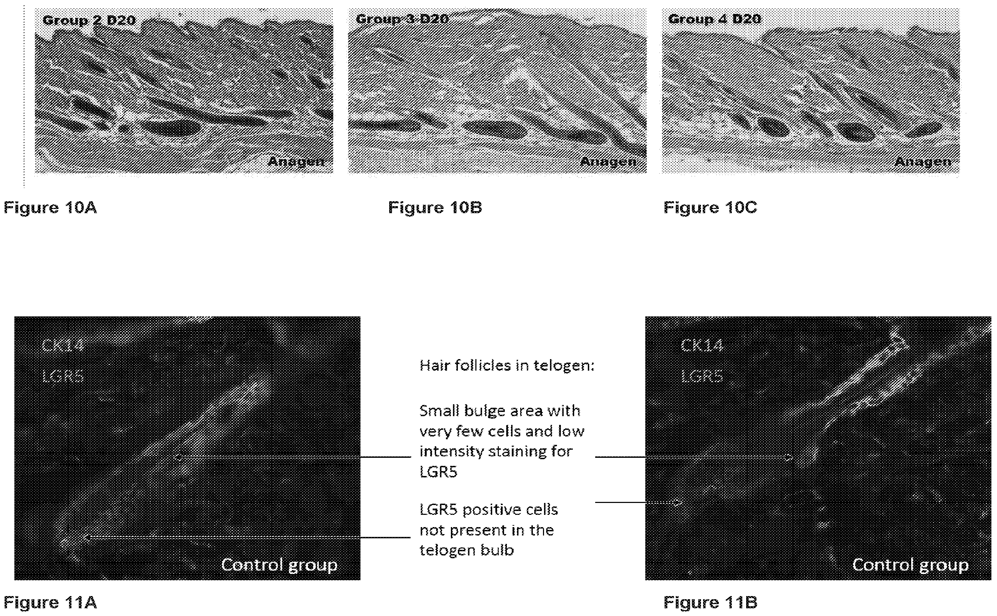

Hematoxylin and eosin staining demonstrates the telogen onset in day 1 and persistence at the end of the study in the control group (Figure 8). The treatment groups display telogen to anagen transition in the areas that do not display yet visible hair growth (Figure 9) and typical anagen hair follicles in the new hair patches (Figure 10).

In telogen phase, the hair follicle stem cells are dormant localized in the bulge area of the old follicle. In the absence of Wnt, LGR5 expression is minimal or absent. CK14 is a skin basal cell marker found throughout the outer root sheath of the hair follicle (Figure 11). During transition to anagen, Wnt signaling stabilizes the β-catenin and induce Lgr5, a putative hair follicle stem cell marker. Lgr5+ cells fuel the actual hair follicle shaft upon migration into the dermal papilla (Figure 12).

SOX9 is a pioneer factor governing hair follicle stem cell fate and plasticity, essential for outer root sheath (ORS) differentiation and the formation of the hair stem cell compartment in the bulge. Sox9 expression depends on sonic hedgehog (Shh) signaling. The treated animals show hair stem cell mobilization by Sox9 positivity (Figure 13).

### Example 3. Ex vivo human hair follicle cultures

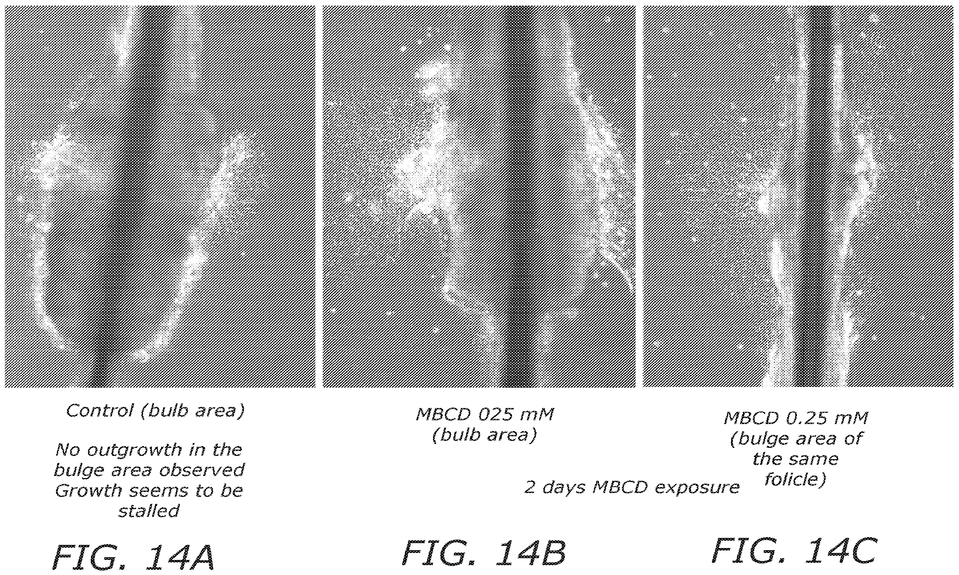

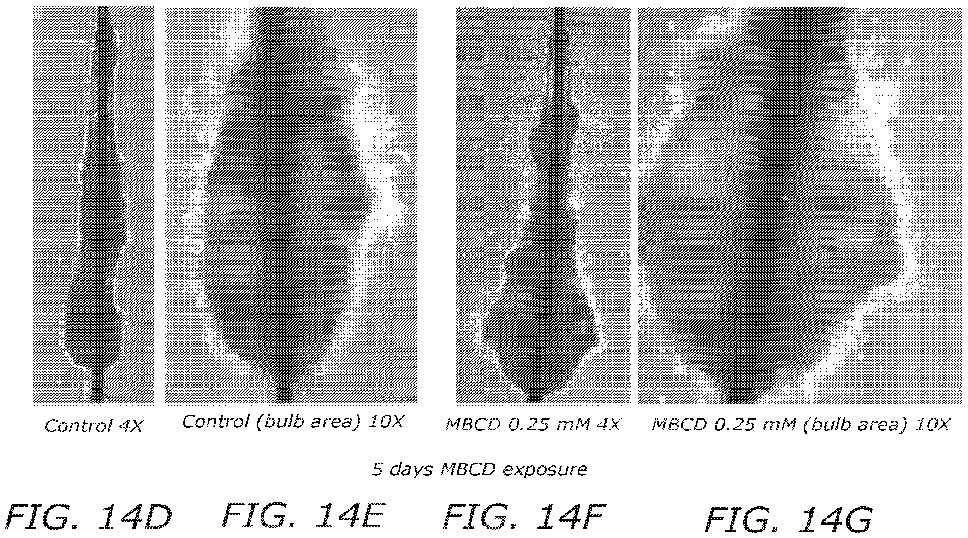

Plucked human hair with evident presence of the bulb area were immersed immediately after collection in cell growth media. Some of the hair samples were exposed to with lipid-modified protein/MBCD complexes (loaded MBCD) from embryonic stem cell culture as described in Example 2 (referred to as active ingredient), or unloaded MBCD as control, at the same concentration of 0.25 mM of the cyclodextrin component. No other growth factors (e.g., EGF, KGF, etc) were used in the hair follicle cultures. After 2 days in culture (Figure 14A-C), attachment and a small outgrowth of cells was observed in both MBCD-containing and control follicle cultures, with more outgrowth in those follicle cultures containing the MBCD-loaded factors. After 5 days of exposure (Figure 14D-G), the control follicles underwent senescence and detached from the substrate, while the follicle cultures exposed to MBCD continued to grow in diameter and adherent cell outgrowth.

Human hair follicles from a non-balding man were dissected out of scalp samples. A total of 15 hair follicles per group were transferred in standard DMEM:F12 with 5% fetal bovine serum culture media and exposed to either the control or the active ingredient at concentrations of 0.1 mM, 0.25 mM or 0.5 mM of the cyclodextrin component. Hair length and hair follicle thickness were measured on days 0 and 7.

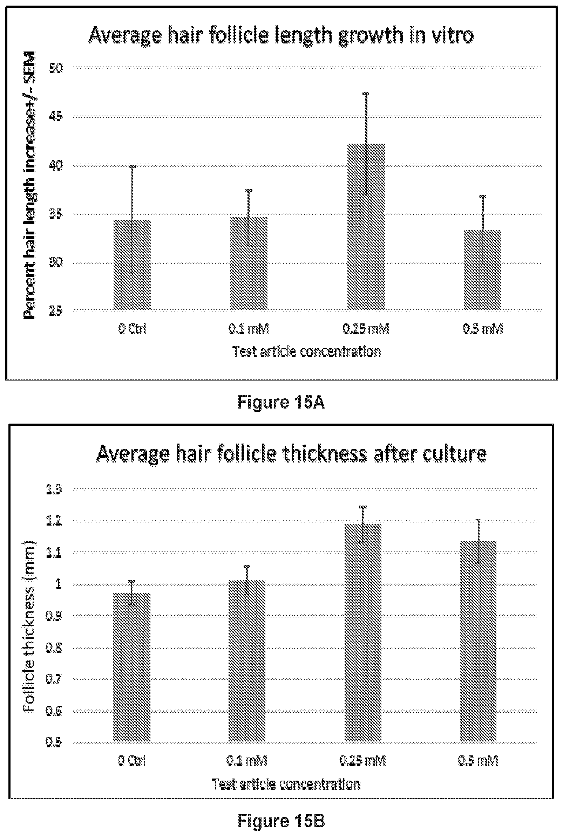

Within 7 days, most of the hair follicles grew in length, resulting in an increase in legnth from 0 to 63%. Hair follicles treated with 0.25 mM active ingredient grew 42% +/-13, while follicles in the other treatment grew 33 to 34%. The same test condition reached statistical significance (p< 0.01) in follicle thickness growth (Figures 15A-B).

### Example 4. Clinical testing of formulation containing cell membrane-bound lipid modified signaling factors

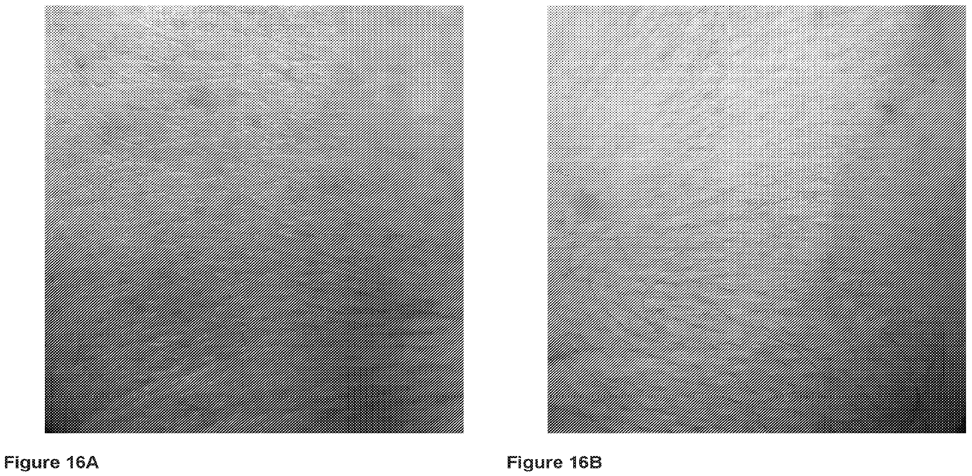

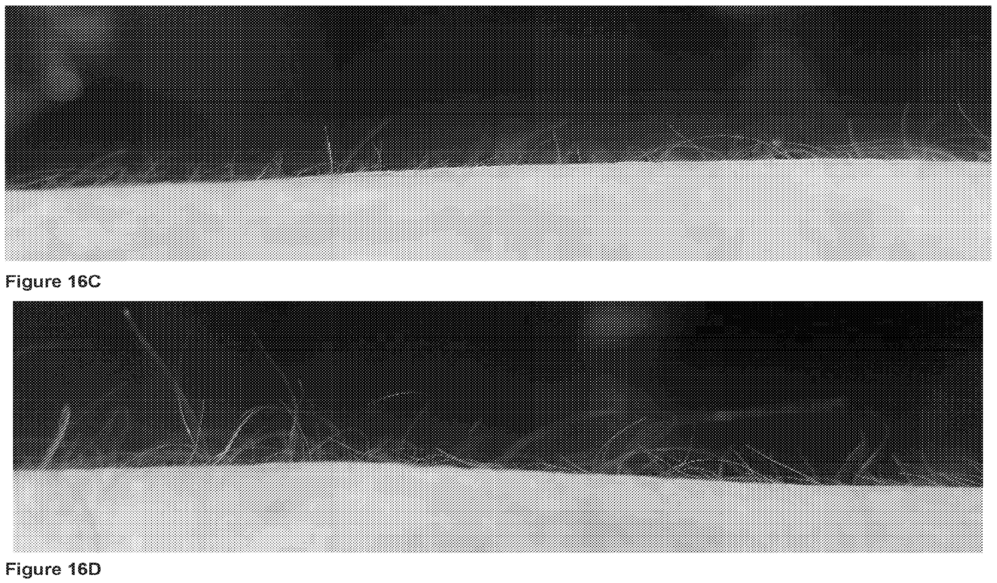

An active ingredient consisting of cyclodextrin/lipid-modified protein complexes was produced by exposing a culture of partial differentiated human embryonic stem cells to a harvest solution containing 10 mM methyl-β-cyclodextrin and 20% trehalose in water for injection, 1 mL/10⁶ cells, for 3 hours at room temperature. The composition was applied externally on the dorsal area of the wrist joint of one hand, while the other hand was left untreated. This section of the hand is covered with terminal arm velus hair with identical left and right pattern and density, however with evident signs of telogen due to mechanical wear. The application consisted of about 1 drop (30 µL) that was spread across the skin, allowed to dry until tackiness, then rubbed until tackiness disappeared. The area was treated daily for 5 days and evaluated after 2 weeks. The growth of the terminal arm hair is clearly accentuated in the treated area (Figure 16C-D). In addition, the age-related wrinkles and spotty pigmentations were clearly reduced, and the skin texture improved. The improvement of the tactile sensation was reported by the subject by increasing the 2-point discriminative ability. The observations suggest a rejuvenating effect on the skin (Figure 16A-B)

Unless otherwise indicated, all numbers expressing quantities of ingredients, properties such as molecular weight, reaction conditions, and so forth used in the specification and claims are to be understood as being modified in all instances by the term "about." As used herein the terms "about" and "approximately" means within 10 to 15%, preferably within 5 to 10%. Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

Furthermore, numerous references have been made to patents and printed publications throughout this specification. Each of the above-cited references and printed publications are individually incorporated herein by reference in their entirety.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The following clauses, describing aspects of the invention, are part of the description
1. A composition comprising a complex of lipid-modified proteins and a cyclodextrin.
2. The composition according to clause 1, wherein the cyclodextrin is one or more of α-cyclodextrin, β-cyclodextrin, γ-cyclodextrin, or methyl-β-cyclodextrin.
3. The composition according to clauses 1 or 2 wherein the cyclodextrin is a chemically modified cyclodextrin, modified by hydrogenation, hydroformylation, methylation, oxidation, reduction, or a carbon-carbon coupling reaction.
4. The composition according to any one of clauses 1-3, wherein the cyclodextrin is methyl-β-cyclodextrin.
5. The composition according to any one of clauses 1-4, wherein the lipid-modified proteins comprise one or more Wingless (Wnt) or Hedgehog (Hh) proteins associated with a cell membrane lipid.
6. The composition according to clause 5, wherein the Hh protein is one or more of a Sonic Hedgehog (Shh) protein, a Desert Hedgehog (Dhh) protein, or an Indian Hedgehog (Ihh) protein.
7. The composition according to clause 5, wherein the Wnt protein is one or more of Wnt3a, Wnt7b, or Wnt1 Ob.
8. The composition according to any one of clauses 1-4, wherein the lipid-modified proteins comprise of other proteins than those belonging to the Wingless (Wnt) or Hedgehog (Hh) families.
9. The composition according to any one of clause 1-8, wherein the lipid-modified proteins are harvested from a population of stem cells.
10. The composition according to clause 9, wherein the stem cells are embryonic stem cells, parthenogenic stem cells, adult stem cells, fetal stem cells, or induced pluripotent stem cells.
11. The composition according to clauses 9 or 10, wherein the stem cells are mammalian stem cells.
12. The composition according to clauses 9 or 11, wherein the stem cells are human stem cells.
13. The composition according to any one of clauses 9-12, wherein the stem cells are genetically engineered to overexpress Wnt or Hh proteins.
14. The composition according to any one of clauses 9-13, wherein the stem cells are generically engineered to be immortal.
15. The composition according to clause 14, wherein the stem cells are genetically engineered to express telomerase reverse transcriptase (hTERT).
16. The composition according to any one of clauses 1-15, further comprising at least one kosmotrope.
17. The composition according to clause 16, wherein the at least one kosmotrope is propylene glycol, proline, trehalose, ectoine, or trimethylamine N-oxide.
18. The composition according to clause 16, wherein the at least one kosmotrope is trehalose.
19. A topical composition comprising the complex of lipid-modified proteins and a cyclodextrin according to any one of clause 1-18.
20. The topical composition according to clauses 19 or 20, wherein the composition is in an aqueous formulation.
21. The topical composition according to clause 19, further comprising at least one kosmotrope, and an antimicrobial agent.
22. The topical composition according to clause 21, wherein the at least one kosmotrope is trehalose.
23. The topical composition according to clause 21, wherein the antimicrobial agent comprises silver particles.
24. The topical composition according to clause 21, wherein the silver particles are silver nanoparticles or silver microparticles.
25. The topical composition according to any one of clauses 19-24, where the pH is between about 4.5 and about 8.0.
26. A method of promoting tissue regeneration in a tissue in need thereof, comprising exposing a tissue to a composition of one of clauses 1-25.
27 A method of promoting tissue rejuvenation in a tissue in need thereof, comprising exposing a tissue to a composition of one of clauses 1-25.
28. A method of restoring sensory nerve function in a tissue, comprising exposing the tissue to a composition of one of clauses 1-25.
29. The method according to any one of clauses 26-28, wherein the tissue is skin.
30. The method according to any one of clauses 26-28, wherein the tissue is scar tissue.
31. A method of promoting hair growth, comprising exposing hair follicles to a composition of one of clauses 1-25.
32. The method according to clause 31, wherein the hair growth is promoted in a subject having senescent alopecia, alopecia totalis, tellogen and anagen effluvium, or alopecia areata.
33. The method according to clauses 31 or 32, wherein the hair follicles are on the scalp of a subject.
34. The method according to clauses 31 or 32, wherein the hair follicles are the eyelashes of a subject.
35. The method according to clauses 31 or 32, wherein the hair follicles are the eyebrows of a subject.
36. The method according to clauses 31 or 32, wherein the hair follicles are on the face of a subject.
37. The method according to clauses 31 or 32, wherein the hair follicles are on the chest of a subject.
38. The method according to clauses 31 or 32, wherein the hair follicles are on the arms of a subject.
39. The method according to clauses 31 or 32, wherein the hair follicles are on the legs of a subject.
40. A method of improving the appearance of skin comprising exposing the skin to a composition of one of clauses 1-24.
41. The method according to clause 40, wherein the appearance improved is one or more of skin texture, wrinkles, discolorations, and age spots.
42. A method of improving the appearance of hair comprising exposing the hair follicles to a composition of one of clauses 1-24.
43. A method of producing the composition of any one of clauses 1-22, comprising:
   culturing stem cells which are capable of producing Wnt and Hh proteins in a culture media;
   incubating the cells in a harvest solution comprising a cyclodextrin to obtain cyclodextrin complexes of lipid-modified proteins; and
   mixing the cyclodextrin/lipid-modified protein complexes with one or more pharmaceutically acceptable excipients to form a topical formulation.
44. The method according to clause 43, wherein the harvest solution further comprises at least one kosmotrope.
45. The method according to clause 43, wherein the harvest solution comprises a stabilizing agent.
46. The method according to clause 45, wherein the stabilizing agent is a kosmotrope
47. The method according to clauses 44 or 46, wherein the kosmotrope is trehalose.
48. The method according to any one of clauses 44, 46, or 47, wherein the concentration of kosmotrope in the harvest solution is about 5% to about 30%.
49. The method according to any one of clauses 44, or 46-48, wherein the concentration of kosmotrope is 20%.
50. The method according to any one of clauses 43-49, wherein the harvest solution comprises an aqueous solution of a cyclodextrin.
51. The method according to any one of clauses 43-50, wherein the cyclodextrin is methyl-β-cyclodextrin.
52. The method according to any one of clauses 43-51, wherein the concentration of cyclodextrin in the harvest solution is about 1 mM to about 20 mM.
53. The method according any one of clauses 43-52, wherein the concentration of cyclodextrin in the harvest solution is about 10 mM.
54. The method according to any one of clauses 43-53, wherein the cyclodextrin/lipid-modified protein complex solution is stored at 4°C or lower.
55. The method according to any one of clauses 43-53, wherein the cyclodextrin/lipid-modified protein complexes solution is lyophilized.
56. The method according to clause 43, wherein the one or more pharmaceutically acceptable excipients comprises one or more preservatives.
57. The method according to clause 43, wherein the one or more pharmaceutically acceptable excipients comprises one or more antimicrobial agents.

## Claims

1. A composition comprising a heterogeneous mixture of complexes, the heterogeneous mixture of complexes comprising:
a first complex comprising a first cyclodextrin molecule and a Wingless (Wnt) protein that is covalently bound to a first cell membrane lipid; and
a second complex comprising a second cyclodextrin molecule and a Hedgehog (Hh) protein that is covalently bound to a second cell membrane lipid,
optionally wherein the heterogenous mixture of complexes is about 5% to 25% of the composition.

2. The composition according to claim 1, wherein the first cyclodextrin molecule or second cyclodextrin molecule is one or more of α-cyclodextrin, β-cyclodextrin, γ-cyclodextrin, or methyl-β-cyclodextrin.

3. The composition according to claim 1 or 2, wherein the first cyclodextrin molecule or second cyclodextrin molecule is a chemically modified cyclodextrin molecule, optionally wherein the first cyclodextrin molecule or the second cyclodextrin molecule is modified by hydrogenation, hydroformylation, methylation, oxidation, reduction, or a carbon-carbon coupling reaction.

4. The composition according to any one of claims 1-3, wherein the first cyclodextrin molecule or the second cyclodextrin molecule is methyl-β-cyclodextrin.

5. The composition according to any one of claims 1-4, wherein the Hh protein is one or more of a Sonic Hedgehog (Shh) protein, a Desert Hedgehog (Dhh) protein, or an Indian Hedgehog (Ihh) protein.

6. The composition according to any one of claims 1-5, wherein the Wnt protein or Hh protein is harvested from a population of stem cells, optionally wherein the population of stem cells comprise embryonic stem cells, parthenogenic stem cells, adult stem cells, fetal stem cells, or induced pluripotent stem cells.

7. The composition according to claim 6, wherein the population of stem cells comprise mammalian stem cells, optionally wherein the mammalian stem cells comprise human stem cells.

8. The composition according to any one of claims 6-7, wherein the population of stem cells are genetically engineered to overexpress Wnt or Hh proteins.

9. The composition according to any one of claims 1-8, wherein the first cell membrane lipid or the second cell membrane lipid comprises myristate, palmitate, palmitoleate, oleate, farnesyl, geranylgeranyl, phosphatidyl-ethanolamine, cholesterol, or glycophosphatidylinositol (GPI) anchor.

10. The composition according to any one of claims 1-9, wherein the composition is a lyophilized composition.

11. The composition according to any one of claims 1-10, wherein the Wnt protein comprises Wnt3a, Wnt7b, or Wnt10b.

12. The composition according to any one of claims 1-11, wherein the composition is a topical composition.

13. The composition according to any one of claims 1-12, further comprising at least one antimicrobial agent.

14. The composition according to claim 13, wherein the antimicrobial agent comprises silver particles, silver nanoparticles, or silver microparticles.

15. The composition according to any one of claims 1-15, where the pH is between 4.5 and 8.0.
